# EUROPÄISCHE PATENTSCHRIFT

(11) **EP 0 569 623 B1**
(45) Veröffentlichungstag und Bekanntmachung des Hinweises auf die Patenterteilung: **09.07.1997**
(21) Anmeldenummer: 92250083.0
(22) Anmeldetag: 13.04.1992
(51) Int. Cl.: C12N 1/04

(54) **Mikroorganismenhaltiges Kulturlyophilisat mit vermehrungsfähigen Keimen, Verfahren zu seiner Herstellung und seine Verwendung**
Microorganisms containing lyophilizate of a culture medium with proliferous germs
Lyophilisat d'un milieu de culture contenant des micro-organismes à germes propageables

(43) Veröffentlichungstag der Anmeldung: 18.11.1993
(73) Patentinhaber: Lauer, Eckhard, Dr., D-12249 Berlin (DE)
(72) Erfinder: Lauer, Eckhard, Dr., D-12249 Berlin (DE)
(74) Vertreter: Wablat, Wolfgang, Dr.Dr.

(56) Entgegenhaltungen:
- DE-C- 4 109 036
- Week 8830, 30. Juni 1988 Derwent Publications Ltd., London, GB; AN 88-211410/30

## Beschreibung

Die Erfindung betrifft ein mikroorganismenhaltiges Kulturlyophilisat mit vermehrungsfähigen Keimen, insbesondere von Keimen der Artengruppen Acidophilus, der Gattung Bifidobacterium der Art Escherichia coli, ein Verfahren zu dessen Herstellung sowie dessen Verwendung.

Seit vielen Jahrzehnten gab es mannigfache Versuche, mit Hilfe von Joghurt oder anderen Sauermilch-Produkten die bakterielle Besiedlung im Darm (Intestinalflora) günstig zu beeinflussen. Der Optimismus ("Bacillus des langen Lebens" nach Metchnikoff, E. (1908): Prolongation of Life, G. Putnam's Sons, New York) erwies sich jedoch als verfrüht, solange weder über die Intestinalflora noch über die stoffwechsel- und immunphysiologischen Zusammenhänge genügend fundierte Kenntnisse vorlagen. Ebensowenig konnten optimistische Prognosen aus Resultaten überzeugen, die auf Produkten mit qualitativ und quantitativ nicht ausreichend definierten Lebendkeimzahlen basierten. Aussagekräftige und vergleichbare Resultate waren so nicht zu erzielen.

Mit dem Fortschritt sowohl der wissenschaftlichen als auch technologischen Erkenntnisse konnten vor allem unter Einsatz von gefriergetrockneten Produkten während der letzten 30 Jahre erhebliche Verbesserungen erzielt werden. Derartige Produkte können heute sowohl als Arzneistoffe als auch für enzymatische, biotechnologische und kosmetische Zwecke bis hin zu umwelttechnischen Prozessen eingesetzt werden.

Seit jeher ist jedoch die Herstellung von Kulturlyophilisaten für Arzneimittel ein besonderes Problem geblieben, da weder dem Apotheker noch dem Verbraucher mit Vakuumlagerung oder Lagerung in der Gefriertruhe gedient ist und diese doch die einzigen Schutzmaßnahmen darstellen, die für den Erhalt der Vermehrungsfähigkeit der Mikroorganismen als relativ sicher angesehen werden. Ohne solchen Schutz sind schädliche Einflüsse durch Feuchte, Temperatur und Luftsauerstoff unvermeidlich und können nur durch geeignete Kombinationen von Hilfs-, Schutz- und Trägerstoffen in der inneren Phase des Lyophilisats mehr oder weniger kompensiert werden.

Als nützlich erwiesen sich bisher Stoffe, wie Kohlenhydrate, Gelatine, Trockenmagermilch und Glutamat, deren Anwendungen sich auch in verschiedenen Patenten niedergeschlagen haben. Die Schutzmechanismen derartig unterschiedlicher Stoffe sind aber bisher ebensowenig bekannt wie allgemein gültige Aussagen über die anzuwendenden Bedingungen bei verschiedenen Mikroorganismen, die sich zusätzlich in ihrer Empfindlichkeit gegenüber den Lagerbedingungen unterscheiden (Morichi, J. (1972): Mechanism and presentation of cellular injury in lactic acid bacteria subjected to freezing and drying, *56th Ann. Session of IDF,* Japan;
Ishibashi, N., et al. (1985): Effect of water activity on the viability of freeze-dried bifidobacteria and lactic acid bacteria. Proceed of Commission C1 Tokyo Meeting, Intern. Institute of Refrigeration, in: *Bibliography of Bifidobacterium and Lactulose,* ed. Morinaga Milk Ind. Co. Ltd., p. 139-144;
Bannikova, L., et al. (1964): Dried cultures from bacterial concentrate. Molochnaya Promyschlennost. 25, 39-41;
Bannikova, L., and I.V. Lagoda (1970): Production of dried starter cultures from bacterial concentrate. *XVIII Int. Dairy Congress* IE, 277;
Lagoda, I.V., and L.A. Bannikova (1970): Some factors affecting quality and storage life of dried starters. Molochnaya Promyschlennost. 31, 11-15;
Collins, E.B., and B.J. Hall (1984): Growth of bifidobacteria in milk and preparation of *Bifidobacterium infantis* for a dietary adjunct. J. Dairy Sci. 67, 1376-1380;
Font de Valdez, G., et al. (1985): Effect of rehydration temperature on the survival of freeze-dried lactic acid bacteria. Milchwissenschaft 40, 147-148; EP 0 159 891, US 4,205,132, US 3,897,307 und SU 1 364 341).

Nicht zuletzt sind Aspekte zur Verträglichkeit und Toxikologie zu beachten.

Aufgabe der Erfindung war es daher, eine geeignete Kombination aus Hilfs- und Schutzstoffen für die innere Phase (Zusatz vor der Gefriertrocknung) zu finden, die nicht nur die Vermehrungsfähigkeit sehr unterschiedlich empfindlicher Mikroorganismen unter relativ einfachen Lagerbedingungen - nicht über 8°C, vor Licht und Feuchte geschützt - ausreichend für mehrere Jahre gewährleistet, sondern zusätzlich auch eine schadlose Verarbeitung derartiger Grundstoffe unter normalen atmosphärischen Raumbedingungen zu handelsgemäßen Produkten erlaubt.

Überraschenderweise wurde nun gefunden, daß eine bestimmte Kombination aus Lactose, Gelatine und Ascorbinsäure diese Aufgabe erfüllt (vgl. Patentanspruch 1).

Bevorzugte Ausführungsformen des mikroorganismenhaltigen Kulturlyophilisats zeichnen sich dadurch aus,
- daß es einen oder mehrere Bakterienstämme oder -arten, insbesondere L. acidophilus, L. crispatus und L. gasseri enthält,
- daß es Zusätze des Substanzgemisches aus Lactose, Gelatine und Ascorbinsäure im Trockengewichtsverhältnis (Kultur/Lactose/Gelatine/Ascorbinsäure) von 2 : 16 : 1 : 0,2 enthält,
- daß es Zusätze des Substanzgemisches aus Lactose, Gelatine und Ascorbinsäure im Trockengewichtsverhältnis (Kultur/Kohlenhydrate/ Gelatine/ Ascorbinsäure) von 8 : 16 : 1 : 0,5 enthält.

Die Variabilität des Gehalts an Ascorbinsäure beruht auf deren unterschiedlicher Schutzwirkung bei verschiedenen bzw. unterschiedlich empfindlichen Mikroorganismen und beruht weitestgehend auf Empirie.

Bei weniger empfindlicheren Bakterienstämmen kommt man auch mit weniger Ascorbinsäure aus als bei sehr empfindlichen Stämmen, d. h. je nach empirisch zu ermittelnder Empfindlichkeit eines Bakterienstammes kann der Ascorbinsäuregehalt niedrig oder hoch sein, im einzelnen 0,1 bis 1,0 im Gewichtsverhältnis.

Eine weitere bevorzugte Ausführungsform der erfindungsgemäßen Kulturlyophilisate besteht darin, daß bei Ersatz von Mikroorganismenkulturen durch gewaschene Zellsuspensionen, die einen geringeren Gehalt an Salzen und anderen Mediumbestandteilen und größere Mengen an Zellen enthalten, eine Kombination aus Zellsuspension, Lactose, Gelatine und Ascorbinsäure im Trockengewichtsverhältnis von 2 : 16 : 1 : 0,2 besteht.

Ein weiterer Gegenstand der Erfindung ist das Verfahren des Patentanspruchs 5.

Ein weiterer Gegenstand der Erfindung ist die Verwendung dieser mikroorganismenhaltigen Kulturlyophilisate zur Stammhaltung als Wirkstoffe in Arzneimitteln.

Durch den Einsatz der Hilfs- bzw. Schutz kombination aus Lactose, Gelatine und Ascorbinsäure werden besonders stabile mikroorganismenhaltige Kulturlyophilisate erhalten. So können mit dieser Hilfsstoffkombination recht unterschiedlich empfindliche Mikroorganismen, wie Escherichia coli, Bifidobakterien und Lactobazillen, ausreichend geschützt werden, so daß nicht nur eine Verarbeitung derartiger Lyophilisate bei normaler Raumtemperatur und Luftfeuchte, sondern auch eine stabile Lagerung bei trockener Lagerung im Kühlschrank (unter 8 °C) für mindestens 3 Jahre möglich ist. Dieses genügt allen Ansprüchen aus Erfordernissen für derartige Arzneistoffe, wie sie eingangs beschrieben wurden.

Besonders vorteilhaft ist, daß über eine Lagerungszeit von wenigstens 3 Jahren unter 8 °C ein Wert von 10 % vom Ausgangswert nicht unterschritten wird.

Durch die nachfolgenden Beispiele wird die Erfindung näher erläutert.

### Beispiel 1:

Kulturverfahren: In einem Kulturmedium, das im wesentlichen aus Voll- oder Magermilch mit geeigneten Zusätzen besteht, wird die zu bearbeitende Stammkultur bis zur stationären Wachstumsphase kultiviert, mit einer Natriumcarbonatlösung (10 %-ig; G/V) neutralisiert (pH: ca. 6,0), mit einer Schutz- und Hilfsstofflösung aus Lactose, Gelatine und Ascorbinsäure vermengt und gefriergetrocknet bis zur Druckstabilität (prakt. konstanter Enddruck).

Schutz- und Hilfsstofflösung: für 2 500 ml Kulturflüssigkeit:

| | |
|---|---|
| a) Lactose: | 800 g Lactose (DAB 9) werden unter Erwärmen in 1 550 ml gereinigtem Wasser (DAB 9) gelöst. |
| | |
| b) Gelatine: | 50 g Gelatine Bloom 200 (DAB 9) werden in der Lösung a) gelöst und diese auf 37 °C temperiert. |
| | |
| c) Ascorbinsäure: | 23 g Ascorbinsäure (DAB 9) werden in 240 ml gereinigtem Wasser (DAB 9) gelöst, mit 5 g Natriumhydroxid neutralisiert (pH: über 6,0) und der Lösung b) zugesetzt. |

Weitere Verarbeitung der Lyophilisate:

Nach dem Lyophilisieren können die erhaltenen Produkte unter normalen Raumbedingungen beispielsweise mit einem Sieb für Trockengranulation, Maschenweite z. B. 0,75 mm, zerkleinert werden. Anschließend können sowohl verschiedene Kulturlyophilisate als auch pulverförmige Hilfsstoffe einfach im Rhönradsystem vermischt und homogenisiert zu Arzneimitteln, z. B. durch Befüllen von Hartgelatine-Steckkapseln, weiterverarbeitet werden. Die Langzeitlagerung erfolgt unter 8 °C vor Feuchtigkeit und Licht geschützt (Tabelle 1).

### Beispiel 2:

Das Beispiel ist völlig analog dem Beispiel 1, nur werden statt 800 g Lactose nur 300 g Lactose und zusätzlich 500 g Trockenmagermilchpulver eingesetzt (Tabelle 1).

### Beispiel 3:

Das Beispiel ist völlig analog dem Beispiel 1, nur werden in der Schutz- und Hilfsstofflösung Ascorbinsäure (siehe c) und Natriumhydroxid weggelassen (Tabelle 2).

### Beispiel 4:

Das Beispiel ist völlig analog dem Beispiel 1, nur wird in der Schutz- und Hilfsstofflösung (siehe c) Gelatine weggelassen (Tabelle 2).

### Beispiel 5:

Das Beispiel ist völlig analog dem Beispiel 1, nur werden in der Schutz- und Hilfsstofflösung (siehe c) Gelatine und Ascorbinsäure (und Natriumhydroxid) weggelassen (Tabelle 1).

### Beispiel 6:

Das Beispiel ist völlig analog dem Beispiel 1, nur werden statt 800 g Lactose und 50 g Gelatine nur 270 g Lactose bzw. 17 g Gelatine eingesetzt (Tabelle 2).

### Beispiel 7:

Das Beispiel ist völlig analog dem Beispiel 1, nur werden wie im Beispiel 6 verringerte Mengen an Lactose und Gelatine eingesetzt und außerdem Ascorbinsäure (und Natriumhydroxid) weggelassen (Tabelle 2).

### Beispiel 8:

Das Beispiel ist völlig analog dem Beispiel 1, nur werden statt 800 g Lactose und 50 g Gelatine die doppelten Mengen, also 1 600 g bzw. 100 g, eingesetzt (Tabelle 3).

### Beispiel 9:

Das Beispiel ist völlig analog dem Beispiel 1, nur werden wie in Beispiel 8 doppelte Mengen an Lactose und Gelatine eingesetzt und außerdem Ascorbinsäure (und Natriumhydroxid) weggelassen (Tabelle 3).

### Beispiel 10:

Das Beispiel ist eine Vermischung des in Beispiel 1 erhaltenen Lyophilisats mit dem in Beispiel 3 erhaltenen Lyophilisat, wobei es sich um verschiedene Bakterienarten handelt. Das Vermischen erfolgt wie im Beispiel 1 unter "Weitere Verarbeitung" angegeben (Tabelle 4).

### Beispiel 11:

Das Beispiel ist analog dem Beispiel 10, nur daß die zu vermischenden Lyophilisate der verschiedenen Bakterienarten ausschließlich mit Ascorbinsäure nach Beispiel 1 hergestellt wurden (Tabelle 4).

### Beispiel 12:

Das Beispiel ist analog dem Beispiel 10, nur daß die zu vermischenden Lyophilisate der verschiedenen Bakterienarten ausschließlich ohne Ascorbinsäure nach Beispiel 3 hergestellt wurden (Tabelle 4).

### Beispiel 13:

Kulturverfahren: In einem geeigneten, flüssigen Kulturmedium, ohne Zusatz von Milch, wird die zu bearbeitende Stammkultur so kultiviert, daß eine optimale Zellsuspension resultiert. Anschließend erfolgt ähnlich wie im Beispiel 1 ein Zusatz einer Schutz- und Hilfsstofflösung aus Lactose, Gelatine und Ascorbinsäure, und dieses Gemisch wird gefriergetrocknet bis zur Druckstabilität (praktisch konstanter Enddruck).

Schutz- und Hilfsstofflösung für ca. 2 500 ml Kulturflüssigkeit:

| | |
|---|---|
| a) Lactose: | 1 200 g Lactose (DAB 9) werden unter Erwärmen in 2 300 ml gereinigtem Wasser (DAB 9) gelöst. |
| | |
| b) Gelatine: | 75 g Gelatine (DAB 9, Bloom 200) werden in der Lösung a) aufgelöst; anschließend wird auf 25 bis 37 °C temperiert. |
| | |
| c) Ascorbinsäure: | 13 g Ascorbinsäure (DAB 9) werden in 250 ml gereinigtem Wasser (DAB 9) gelöst, mit 3 g Natriumhydroxid neutralisiert (pH: über 6,0) und der Lösung b) zugesetzt. |

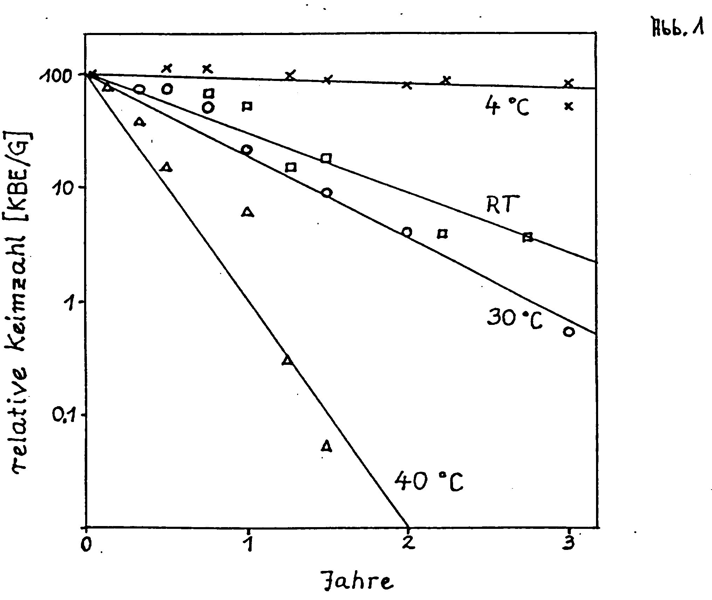

Die Weiterverarbeitung kann wie unter Beispiel 1 beschrieben erfolgen (Abbildung 1).

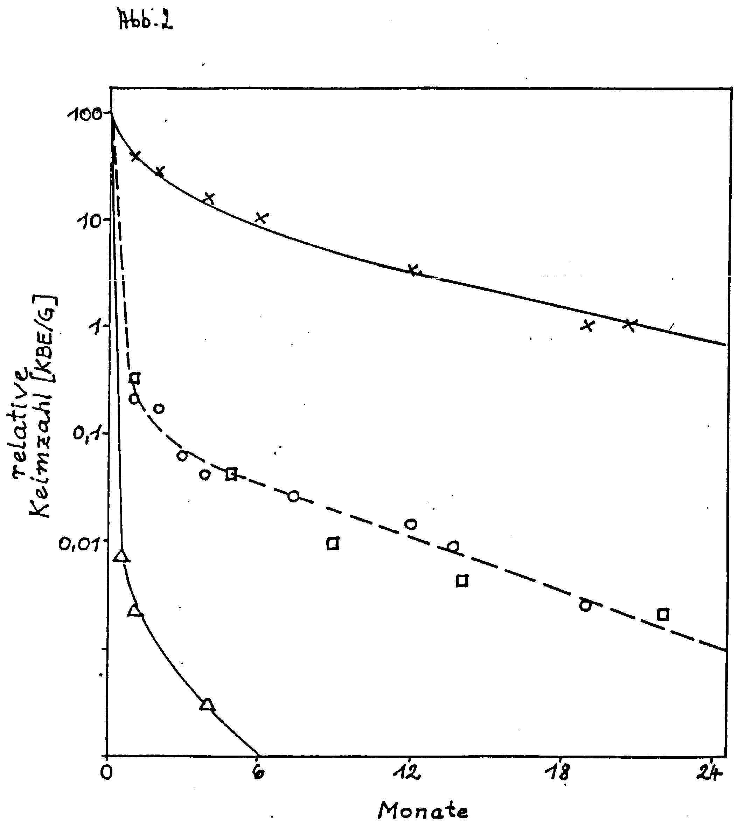

Von den verschiedenen Hilfsstoff-Kombinationen, dargestellt in den Beispielen 1 bis 12, ist eindeutig die Kombination aus Lactose mit Gelatine und Ascorbinsäure am besten. Dies wird in der Abb. 2 anhand von E. coli, gelagert bei Raumtemperatur, noch einmal vergleichend deutlich gemacht.

Als Maß für den Keimgehalt der Proben, der Vermehrungsfähigkeit der Mikroorganismen, gilt - wie in der Mikrobiologie allgemein üblich - die Anzahl der Kolonie bildenden Einheiten (KBE/g), die in Form von Kolonien auf geeigneten, festen Nährböden nach Beimpfen mit verdünnten Kulturproben (Verdünnungsreihen) und nach geeigneter Bebrütung gezählt werden. Alle derartigen Verfahren sind bekanntlich durch eine relativ hohe Ungenauigkeit charakterisiert. Durch die Auftragung des Keimgehalts im logarithmischen Maßstab wird jedoch sowohl den exponentiellen Absterberaten der Bakterien Rechnung getragen als auch eine - trotz der hohen Schwankungsbreite der Gehaltsbestimmung - reproduzierbare Aussage ermöglicht.

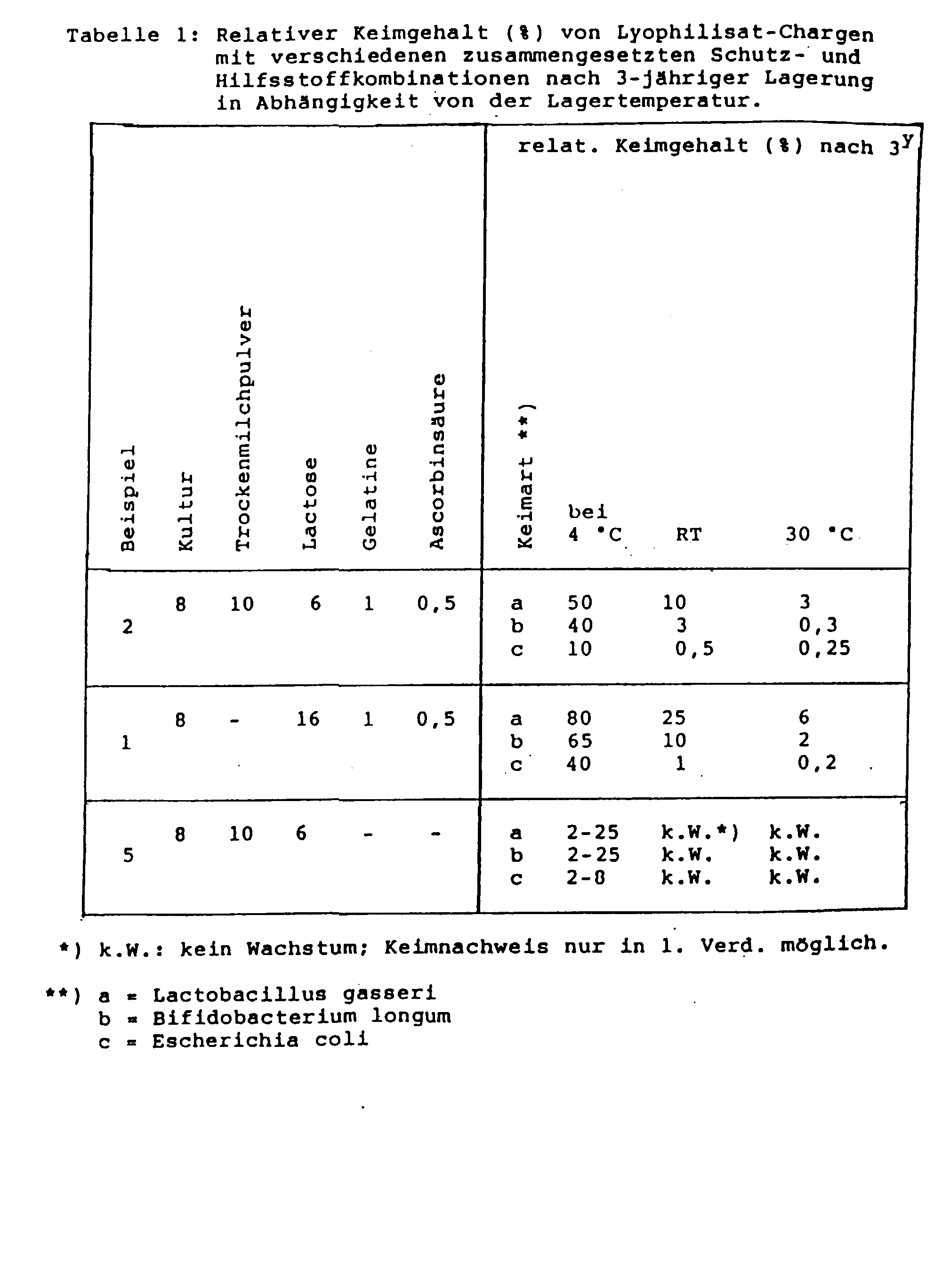

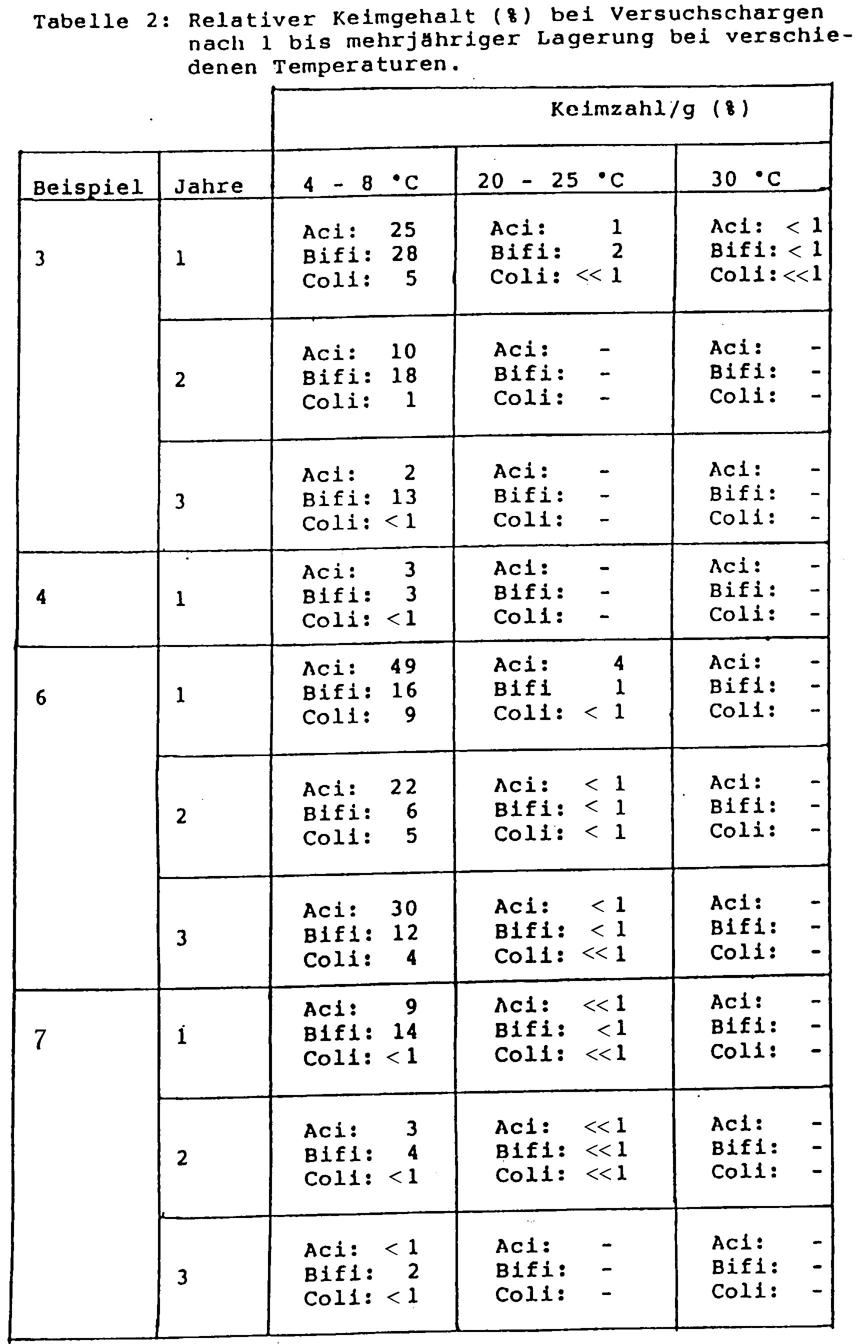

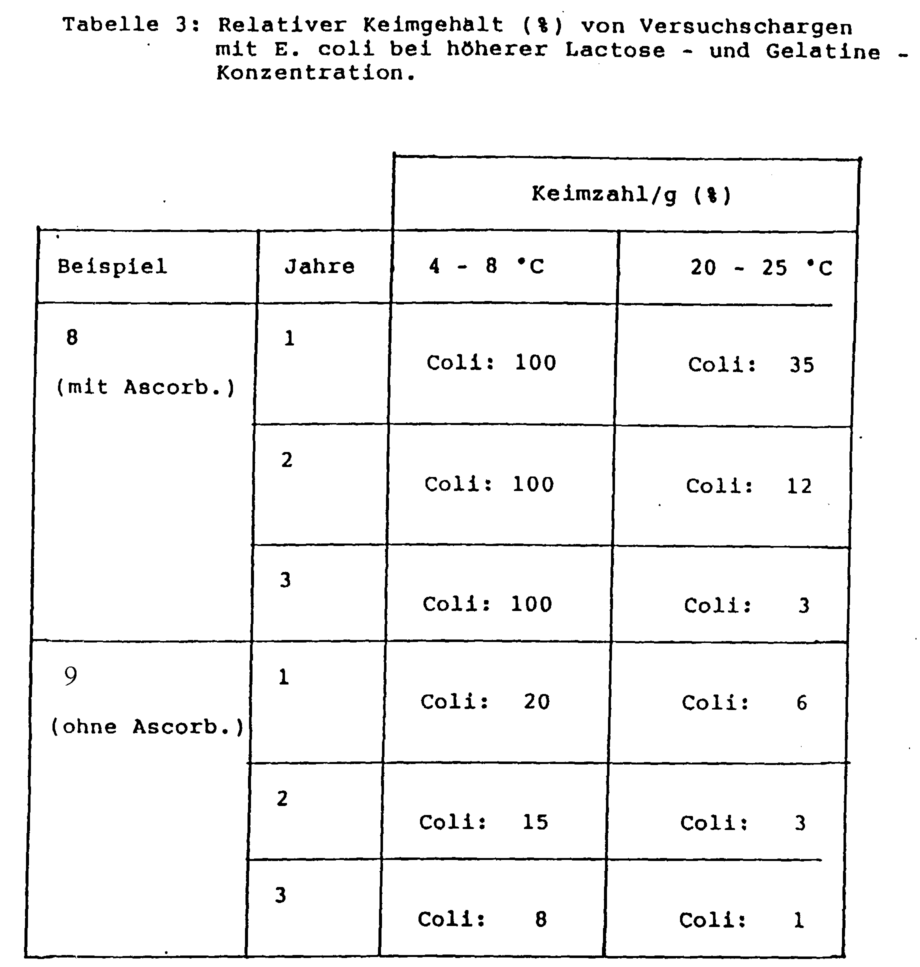

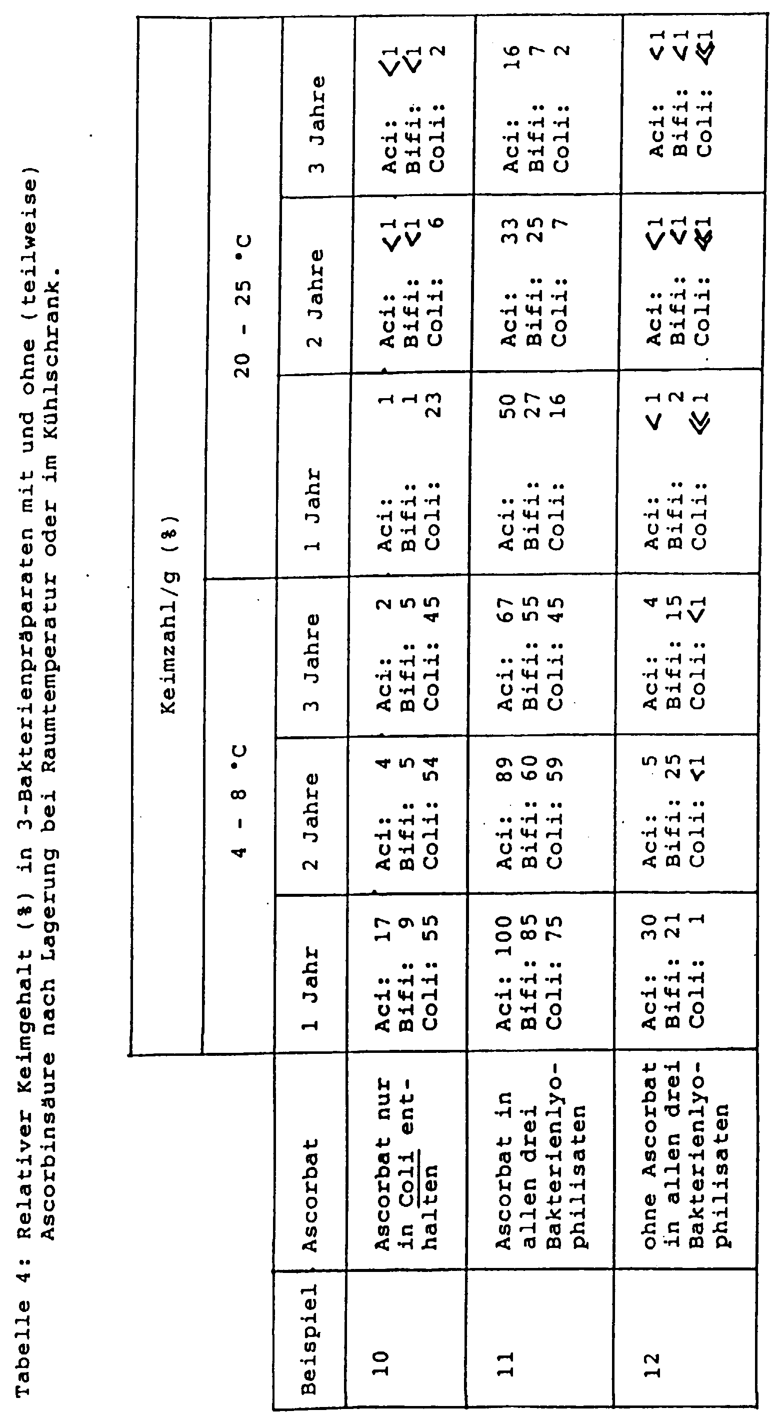

### Abbildung 1 zum Beispiel 13:

Einfluß verschiedener Lagertemperaturen auf die Stabilität der Vermehrungsfähigkeit (KBE/G) von Lactobacillus gasseri nach Gefriertrocknung einer mikrofiltrierten Zellsuspension. Die geprüfte Präparation bestand aus einer Darreichungsform in Hartgelatine-Steckkapseln, Größe 1, mit 200 mg Lyophilisat als Füllung.

### Abbildung 2:

Einfluß verschiedener Schutzstoffkombinationen auf die Stabilität der Vermehrungsfähigkeit von Escherichia coli in Kulturlyophilisaten gelagert bei Raumtemperatur.

Kombinationen:

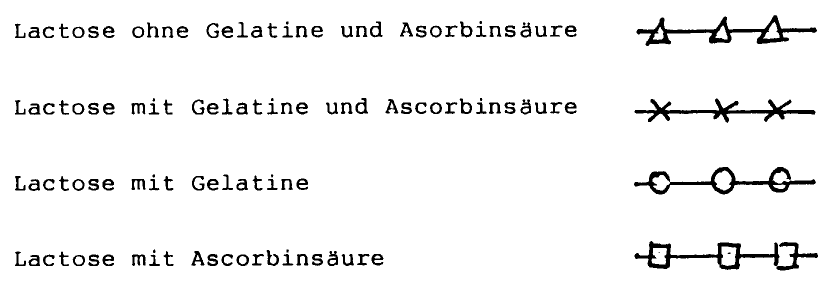

## Patentansprüche

1. Mikroorganismenhaltiges Kulturlyophilisat mit vermehrungsfähigen Keimen, insbesondere von Keimen der Artengruppe Acidophilus, der Gattung Bifidobacterium und der Art Escherichia coli, dadurch gekennzeichnet, daß es zur Erhaltung der Vermehrungsfähigkeit, gemessen als Stabilität des Koloniebildungsvermögen (KBE) auf geeigneten Nährböden, ein Substanzgemisch aus Lactose, Gelatine und Ascorbinsäure im Trockengewichtsverhältnis von 16:1:0,1 bis 16:1:1 in der inneren Phase (Zusatz vor Gefriertrocknung) enthält.

2. Mikroorganismenhaltiges Kulturlyophilisat nach Anspruch 1, dadurch gekennzeichnet, daß es das Substanzgemisch aus Kultur, Lactose, Gelatine und Ascorbinsäure im Trockengewichtsverhältnis von 8:16:1:0,5 enthält.

3. Mikroorganismenhaltiges Kulturlyophilisat nach Anspruch 1 oder 2, dadurch gekennzeichnet, daß es das Substanzgemisch aus Kultur, Lactose, Gelatine und Ascorbinsäure im Trockengewichtsverhältnis von 2:16:1:0,2 enthält.

4. Mikroorganismenhaltiges Kulturlyophilisat nach Anspruch 1 zur Anwendung als Arzneimittel.

5. Verfahren zur Herstellung eines mikroorganismenhaltigen Kulturlyophilisates gemäß Anspruch 1, dadurch gekennzeichnet, daß man die Mikroorganismenkulturen- oder -suspensionen in Abhängigkeit von der stammspezifischen Empfindlichkeit mit Zusätzen aus wäßrigen Lösungen eines Substanzgemisches aus Lactose, Gelatine und Ascorbinsäure im Trockengewichtsverhältnis von 16:1:0,1 bis 16:1:1 in der inneren Phase (Zusatz vor Gefriertrocknung) vermischt und anschließend gefriertrocknet.

6. Verfahren nach Anspruch 5, dadurch gekennzeichnet, daß man das Substanzgemisch aus Kultur, Lactose, Gelatine und Ascorbinsäure im Trockengewichtsverhältnis von 8:16:1:0,5 vermischt.

7. Verfahren nach Anspruch 5 und 6, dadurch gekennzeichnet, daß man das Substanzgemisch aus Kultur, Lactose, Gelatine und Ascorbinsäure im Trockengewichtsverhältnis von 2:16:1:0,2 vermischt.

## Claims

1. Microorganism-containing culture lyophilizate with viable and reproductive germs, especially with germs7X5 of the Acidophilus group of species, of the genus Bifidobacterium and of the species Escherichia coli, characterized in that it contains for preservation of reproduction capability, measured as stability of colony forming ability (colony forming units CFU) on suitable growth media, a mixture of lactose, gelatin and ascorbic acid, with a dry weight ratio between 16 : 1 : 0.1 and 16 : 1 : 1 in the inner phase (addition prior to lyophilization).

2. Microorganism-containing culture lyophilizate according to claim 1, characterized in that it contains the mixture of culture, lactose, gelatin and ascorbic acid in a dry weight ratio of 8 : 16 : 1 : 0.5.

3. Microorganism-containing culture lyophilizate according to claim 1 or 2, characterized in that it contains the mixture of culture, lactose, gelatin and ascorbic acid in a dry weight ratio of 2 : 16 : 1 : 0.2.

4. Microorganism-containing culture lyophilizate according to claim 1 for the use as medical drug.

5. Process for production of a microorganism-containing culture lyophilizate according to claim 1, characterized in that the cultures or suspensions of microorganisms are mixed with additives of aqueous solutions of a mixture of lactose, gelatin and ascorbic acid, the dry weight ratios being between 16 : 1 : 0.1 and 16 : 1 : 1 in the inner phase (addition prior to lyophilization), the mixing being carried through depending on strain-specific sensitivity, and said cultures or suspensions being subsequently lyophilized.

6. Process according to claim 5, characterized in that the mixture of culture, lactose, gelatin and ascorbic acid is mixed in a dry weight ratio of 8 : 16 : 1 : 0.5.

7. Process according to claim 5 or 6, characterized in that the mixture of culture, lactose, gelatin and ascorbic acid is mixed in a dry weight ratio of 2 : 16 : 1 : 0.2.

## Revendications

1. Lyophilisat d'un milieu de culture contenant des micro-organismes à germes prolifères, en particulier issus de germes de l'espèce Acidophilus, du genre Bifidobacterium et du type Escherichia coli, caractérisé en ce qu'il contient un mélange de substances constitué de lactose, de gélatine et d'acide ascorbique dans un rapport pondéral en matière sèche de 16:1:0,1 à 16:1:1 dans la phase interne (addition avant lyophilisation), pour le maintien de la prolifération, tel que mesuré d'après la stabilité du pouvoir de formation de colonies (PFC) dans un bouillon de culture approprié.

2. Lyophilisat d'un milieu de culture contenant des micro-organismes selon la revendication 1, caractérisé en ce qu'il contient le mélange de substances constitué d'un milieu de culture, de lactose, de gélatine et d'acide ascorbique dans un rapport pondéral en matière sèche de 8:16:1:0,5.

3. Lyophilisat d'un milieu de culture contenant des micro-organismes selon la revendication 1 ou la revendication 2, caractérisé en ce qu'il contient le mélange de substances constitué d'un milieu de culture, de lactose, de gélatine et d'acide ascorbique dans un rapport pondéral en matière sèche de 2:16:1:0,2.

4. Lyophilisat d'un milieu de culture contenant des micro-organismes selon la revendication 1 pour l'utilisation en tant que médicament.

5. Procédé pour la préparation d'un lyophilisat d'un milieu de culture contenant des micro-organismes selon la revendication 1, caractérisé en ce que l'on mélange les milieux de culture de micro-organismes ou les suspensions de micro-organismes en fonction de la sensibilité spécifique de la souche considérée avec des produits ajoutés constitués de solutions aqueuses d'un mélange de substances constitué de lactose, de gélatine et d'acide ascorbique dans un rapport pondéral en matière sèche de 16:1:0,1 à 16:1:1 dans la phase interne (addition avant lyophilisation) et en ce qu'on procède ensuite à la lyophilisation.

6. Procédé selon la revendication 5, caractérisé en ce que l'on mélange l'association de substances constitué d'un milieu de culture, de lactose, de gélatine et d'acide ascorbique dans un rapport pondéral en matière sèche de 8:16:1:0,5.

7. Procédé selon les revendications 5 et 6, caractérisé en ce que l'on mélange l'association de substances constitué d'un milieu de culture, de lactose, de gélatine et d'acide ascorbique dans un rapport pondéral en matière sèche de 2:16:1:0,2.
